# FASCICULE DE BREVET EUROPEEN

(11) **EP 2 566 838 B1**
(45) Date de publication et mention de la délivrance du brevet: **02.07.2014**
(21) Numéro de dépôt: 11724462.4
(22) Date de dépôt: 04.05.2011
(51) Int. Cl.: C07C 33/14, C07C 403/08, A61K 8/34, A61Q 13/00, A61Q 19/10, C07C 31/13, C11B 9/00

(54) **COMPOSÉS À LA NOTE BOISÉE**
VERBINDUNGEN MIT EINER HOLZIGEN NOTE
COMPOUNDS WITH A WOODY NOTE

(30) Priorité: 05.05.2010 FR 1001938
(43) Date de publication de la demande: 13.03.2013
(73) Titulaire: V. Mane Fils, 06620 Bar sur Loup (FR)
(72) Inventeur: MURATORE, Agnès, 06560 Valbonne (FR); CHANOT, Jean-Jacques, 06530 Speracedes (FR)
(74) Mandataire: Müllejans, Inge
(86) Numéro de dépôt international: PCT/IB2011/051976
(87) Numéro de publication internationale: WO 2011/138747

(56) Documents cités:
- FR-A1- 2 008 167
- FR-A1- 2 259 091
- US-A- 5 234 902
- US-A1- 2008 039 360
- US-B1- 7 563 925
- FRACKOWIAK ET AL: "Stereochemistry of terpene derivatives. Part 4: Fragrant terpenoid derivatives with an unsaturated gem-dimethylbicyclo[3.1.0]hexane system", TETRAHEDRON ASYMMETRY, PERGAMON PRESS LTD, OXFORD, GB, vol. 16, no. 20, 17 octobre 2005 (2005-10-17), pages 3352-3360, XP005149738, ISSN: 0957-4166, DOI: DOI:10.1016/J.TETASY.2005.09.004
- HASHIDOKO Y ET AL: "Bisabolane sesquiterpenes and a 2-phenoxychromone from rosa Woodsii leaves", PHYTOCHEMISTRY, PERGAMON PRESS, GB, vol. 31, no. 6, 1 juin 1992 (1992-06-01), pages 2148-2149, XP026633446, ISSN: 0031-9422, DOI: DOI:10.1016/0031-9422(92)80384-Q [extrait le 1992-06-01]

## Description

L'invention a trait à la chimie des fragrances et au domaine de la parfumerie. Elle concerne plus particulièrement une nouvelle famille de composés à la note boisée, et s'étend à l'utilisation de ces composés en parfumerie.

En vue d'accroître la gamme des notes offertes aux parfumeurs pour leurs créations, l'industrie de la parfumerie est en perpétuelle recherche de nouveaux composés odorants.

Les composés à la note boisée sont aujourd'hui abondants. A titre d'exemples, nous pouvons citer, notamment :
- des dérivés du norbornane ou du norbornène, comme ceux décrits dans US 4 229 600, US 4 524 017, WO 2003/035595, ou encore
- des dérivés de la décalone, décrits notamment dans WO 2007/031904, US 4 387 048 et US 5 114 915.

Nous pouvons aussi citer FR 2 259 091 qui, au moyen d'une formule générale particulière, désigne une famille de composés revendiqués comme se distinguant par "leur note fruitée et tout à la fois boisée, verte et huileuse". Dans cette famille, on compte notamment les cétones suivantes :
1-((3,3-diméthyl-cyclohexyl)-pent-4-èn-1-one ;
1-((3,3-diméthyl-cyclohex-1-ènyl)-pent-4-èn-1-one ;
1-((3,3-diméthyl-cyclohex-6-ényl)-pent-4-èn-1-one ;
1-((3,3-diméthyl-cyclohex-1-ènyl)-hex-4-èn-1-one ;
1-((3,3-diméthyl-cyclohex-6-ènyl)-hex-4-èn-1-one ;
1-((3,3-diméthyl-cyclohex-6-ènyl)-2-méthyl-but-3-èn-1-one.

De façon apparemment fortuite, ce document (schéma VII) décrit également la synthèse d'alcools secondaires particuliers, tels que les 1-(3,3-diméthylcyclohex-1-ényl)-pent-4-ènol et 1-(3,3-diméthylcyclohex-6-ènyl)-pent-4-ènol, qui pourtant ne répondent pas à la formule générale précédemment évoquée des composés ayant une "note fruitée et tout à la fois boisée, verte et huileuse". Ce document est d'ailleurs silencieux quant à l'odeur qu'ils dégagent. Après vérification, les inventeurs n'ont pas constaté de note boisée, mais une odeur de faible intensité, plutôt verte, fruitée, d'ananas.

Malgré un nombre significatif de composés à la note boisée déjà existants, il subsiste un besoin pour de nouvelles nuances dans les odeurs boisées. Au-delà de cet objectif premier, l'invention vise également à proposer des composés qui soient facilement accessibles grâce à une fabrication simple et compatible avec les exigences de l'industrie, et qui, de par une stabilité appréciable, peuvent être utilisés en parfumerie, dans une large gamme d'applications.

Le terme "parfumerie" est ici utilisé dans son sens général ; il désigne non seulement la parfumerie traditionnelle (alcoolique ou non), mais également les autres domaines dans lesquels l'odeur des produits est importante. Il peut être ainsi fait référence à des compositions de parfumerie au sens habituel et traditionel (telles que bases et concentrés parfumants, parfums, eaux de Cologne, eaux de toilette, désodorisants d'intérieur, parfums d'ambiance, bougie parfumées et produits similaires), à des compositions topiques (en particulier cosmétiques, telles que crèmes pour le visage et/ou le corps, poudres de talc, huiles pour cheveux, shampooings, lotions capillaires, sels et huiles de bain, gels de douche et/ou de bain, savons de toilette, antiperspirants et déodorants corporels, lotions et crèmes de rasage, savons, dentifrices, bains de bouche, pommades, et produits similaires), ainsi qu'à des produits d'entretien, notamment ménagers (tels que détergents, lessives, assouplissants, désodorisants d'intérieur, parfums d'ambiance et produits similaires).

L'invention a ainsi pour objet des composés de formule générale ci-après : 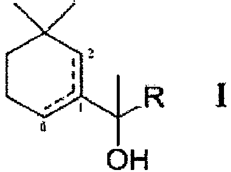 dans laquelle :
- le cycle à 6 atomes de carbone est saturé ou présente une double liaison entre les carbones C1 et C2 ou entre les carbones C1 et C6,
- R est choisi parmi un groupe alkyle en C2-C5 ou alcényle en C2-C5.

Les composés répondant à cette formule (I) dégagent tous des notes boisées plus ou moins subtiles ou prononcées, enrichies de notes plus ou moins florales, fruitées, vertes et/ou animales.

Également, ils présentent l'avantage de pouvoir être obtenus de façon relativement simple, à partir de matières premières facilement accessibles, à savoir le déhydrolinalol (DE 1643710) ou certains de ses dérivés particuliers, comme le déhydroherbac (US 4,264,467) et l'herbac. Le Schéma 1 ci-après en résume les étapes essentielles.

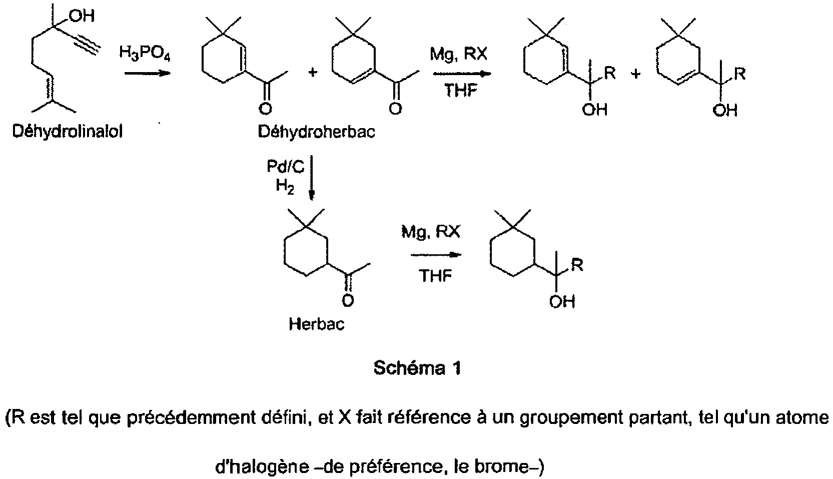

De manière consacrée, la dénomination « déhydroherbac » désigne un mélange des isomères 1-(3,3-diméthylcyclohex-1-ényl)éthanone et 1-(3,3-diméthylcylohex-6-ényl)éthanone ; peu importe la proportion relative entre ces isomères.

Des formes pures de ces isomères peuvent être obtenues à partir du déhydroherbac, grâce à des méthodes de séparation bien connues, comme la séparation par cristallisation et/ou par chromatographie. Elles peuvent aussi être obtenues sélectivement par synthèse, comme cela est décrit notamment dans Z. Chem. (1969) : 9, 64 et dans US 4,264,467.

Tout comme le déhydroherbac, la 1-(3,3-diméthylcyclohex-1-ényl)éthanone et la 1-(3,3-diméthylcylohex-6-ényl)éthanone, dans leur forme pure, peuvent être utilisées comme produits de départ pour la préparation des composés de formule (I) de l'invention.

Au sens de la présente invention, le terme « alkyle en C2-C5 » désigne tout radical monovalent dérivé d'une chaîne carbonée saturée, linéaire ou branchée, contenant 2 à 5 atomes de carbone, notamment les groupes, éthyle, n-propyle, i-propyle, n-butyle, i-butyle, t-butyle et pentyle, 1 (ou 2)-méthylpropyle, 1,2-diméthylpropyle, 1,1-diméthylpropyle, 1(ou 2 ou 3)-méthylbutyle...

De même, le terme « alcényle en C₂-C₅ » désigne tout radical monovalent dérivé d'une chaîne carbonée, linéaire ou branchée, contenant 2 à 5 atomes de carbone et comportant une double liaison, notamment les groupes éthényle, prop-1-ényle, prop-2-ényle, but-1-ényle, but-2-ényle, but-3-ényle, pent-1-ényle, pent-2-ényle, pent-3-ényle, pent-4-ényle, 2-méthylprop-2-ényle, 2,3-diméthylprop-1-ényle, 3-méthylbut-3-ényle... Les alcényles contenant 3 à 5 atomes de carbone (alcényles en C₃-C₅) sont néanmoins préférés, en particulier les groupes prop-2-ényle, but-3-ényle, but-2-ényle, pent-4-ényle, pent-3-ényle et pent-2-ényle.

Dans un premier mode de réalisation, l'invention concerne des composés de formule (I), comme définie précédemment, et pour lesquels le cycle à 6 atomes de carbone est saturé.

Selon un deuxième mode de réalisation, l'invention concerne des composés de formule (I), comme définie précédemment, et pour lesquels le cycle à 6 atomes de carbone présente une double liaison entre les carbones C1 et C2 ou entre les carbones C1 et C6.

Dans les deux cas, avantageusement et selon l'invention, R est choisi parmi les groupes : -CH₂CH₃, -CH₂CH₂=CH₂, -CH₂CH₂CH₃, -CH₂CH₂CH₂CH₃, -CH₂CH(CH₃)₂, -CH(CH₃)CH₂CH₃ et -CH₂CH₂CH(CH₃)₂.

En particulier, les composés préférés de l'invention sont choisis parmi :
- 2-(3,3-diméthylcyclohex-1-ényl)butan-2-ol
- 2-(5,5-diméthylcyclohex-1-ényl)butan-2-ol
- 2-(3,3-diméthylcyclohex-1-ényl)pent-4-én-2-ol
- 2-(5,5-diméthylcyclohex-1-ényl)pent-4-én-2-ol
- 2-(3,3-diméthyleyclohex-1-ényl)pentan-2-ol
- 2-(5,5-diméthylcyclohex-1-ényl)pentan-2-ol
- 2-(3,3-diméthylcyclohex-1-ényl)hexan-2-ol
- 2-(5,5-diméthylcyclohex-1-ényl)hexan-2-ol
- 2-(3,3-diméthylcyclohex-1-ényl)-4-méthylpentan-2-ol
- 2-(5,5-diméthylcyclohex-1-ényl)-4-méthylpentan-2-ol
- 2-(3,3-diméthylcyclohex-1-ényl)-3-méthylpentan-2-ol
- 2-(5,5-diméthylcyclohex-1-ényl)-3-méthylpentan-2-ol
- 2-(3,3-diméthylcyclohex-1-ényl)-5-méthylhexan-2-ol
- 2-(5,5-diméthylcyclohex-1-ényl)-5-méthylhexan-2-ol
- 2-(3,3-diméthylcyclohexyl)butan-2-ol
- 2-(3,3-diméthylcyclohexyl)pent-4-én-2-ol
- 2-(3,3-diméthylcyclohexyl)pentan-2-ol
- 2-(3,3-diméthylcyclohexyl)hexan-2-ol
- 2-(3,3-diméthylcyclohexyl)-4-méthylpentan-2-ol
- 2-(3,3-diméthylcyclohexyl)-5-méthylhexan-2-ol

De par l'odeur plaisante qu'ils dégagent, les composés de l'invention trouvent nombre d'applications en parfumerie, notamment pour la préparation de parfums traditionnels, de compositions cosmétiques et de produits d'entretien.

L'invention concerne ainsi également l'utilisation des composés de l'invention pour la préparation d'une composition odorante ou d'un article odorant dans les applications décrites ci-dessus et/ou ci-après, en particulier en parfumerie, en cosmétique, et pour la préparation de produits d'entretien notamment ménagers.

L'invention s'étend ainsi à des compositions parfumées comprenant au moins un composé de l'invention. Il peut notamment s'agir de compositions de la parfumerie traditionnelle, des compostions cosmétiques, des produits d'entretien, ou encore des "compositions dites intermédiaires", destinées à être utilisées pour la préparation de compositions ou de produits finis (notamment parfums, produits cosmétiques, produits d'entretien).

De telles compositions parfumées sont généralement préparées à partir d'un produit de base, dans lequel le ou les composés de l'invention se trouvent être incorporés. Le produit de base sera aisément déterminé par l'homme du métier en fonction de la composition envisagée et donc de l'utilisation envisagée. La composition de ces produits de base et la nature de leurs composants habituels, tels que solvant(s) et/ou adjuvant(s), sont bien connues de l'homme du métier.

Les composés entrant dans ces compositions parfumées, notamment les composés de l'invention, peuvent être incorporés dans ou sur un matériau support inerte. Les matériaux supports pouvant êtres employés sont nombreux et variés, par exemple des solvants polaires, des huiles, des graisses, des solides finement divisés, des cyclodextrines, des maltodextrines, des gommes, des résines et n'importe quel autre matériau support connu pour de telles compositions (par exemple, savons, bougies, pommades, textiles, lingettes, gels parfumés...).

La quantité efficace des composés de l'invention à incorporer dans ces compositions est fonction de la nature desdites compositions, de l'effet odorant souhaité et de la nature des autres composés odorants éventuellement présents. Elle est aisément déterminée par l'homme du métier, et peut varier dans une plage très étendue, de 0,1 à 99%, en particulier 0,1 à 50%, notamment 0,1 à 30%. Les pourcentages précédents sont exprimés en poids total de la composition.

L'invention concerne en particulier une composition traditionnelle de parfum (notamment une base ou un concentré parfumant, une eau de Cologne, une eau de toilette, un parfum...) comprenant au moins un composé de l'invention ou une composition (par exemple, une composition dite intermédiaire) comprenant elle-même au moins un composé de l'invention.

Au sens de la présente demande, il y a lieu de noter que les expressions « composition de parfum » ou « composition parfumée » sont employées ici de façon interchangeables.

L'invention concerne également en particulier une composition cosmétique (notamment, crème pour le visage et/ou le corps, poudre de talc, huile pour cheveux ou pour le corps, shampooing, lotion capillaire, sels de bain, huile de bain, gel de douche, gel de bain, savon, antiperspirant, déodorant, lotion, crème de rasage, savon de rasage, pâte dentifrice, pommade...) comprenant au moins un composé de l'invention ou une composition (par exemple, une composition dite intermédiaire) comprenant elle-même au moins un composé de l'invention.

L'invention concerne encore un produit d'entretien (notamment, assouplissant, détergent, lessive, désodorisant, parfum d'ambiance...) comprenant au moins un composé de l'invention ou au moins une composition (par exemple, une composition dite intermédiaire) comprenant elle-même au moins un composé de l'invention.

La présence de centres d'asymétrie dans la structure des composés de formule (I) selon l'invention provoque l'existence, pour chacun d'entre eux, de plusieurs formes énantiomériques et/ou diastéréomériques. L'invention couvre également les composés représentés par la formule générale (I) sous forme de mélanges des énantiomères et/ou diastéréomériques, en proportions variables, en particulier les mélanges racémiques. L'invention comprend également les composés de formule (I) sous forme d'un seul énantiomère et/ou diastéréomère. Des mélanges d'énantiomères/diastéréomères ou des formes pures peuvent être obtenus par synthèse à partir de produits de départ optiquement enrichis ou optiquement purs, ou au moyen de méthodes de séparation par cristallisation ou chromatographie.

Les exemples suivants illustrent une manière particulière de préparer les composés de l'invention, ainsi que le profil olfactif de chacun des composés exemplifiés. Ces exemples ne sont donnés que dans un but d'illustration et ne doivent pas être compris comme limitant la portée générale de l'invention.

### Exemple 1 : Préparation des 2-(3,3-diméthylcyclohex-1-ényl)butan-2-ol et 2-(5,5-diméthylcyclohex-1-ényl)butan-2-ol

On place sous azote dans un ballon tricol 1,2 éq. de magnésium recouvert de THF. Sont coulés goutte à goutte 1,2 éq. de bromoéthane. La température s'élève jusqu'au reflux. Lorsque le magnésium est entièrement consommé, le milieu réactionnel est placé à 5-10 °C et 1 éq. de déhydroherbac est coulé goutte à goutte. Le mélange est ensuite agité à température ambiante pendant deux heures, puis versé sur un mélange HCl 10% aq. / glace (50:50). Après une dizaine de minutes d'agitation, les phases sont séparées. La phase aqueuse est extraite deux fois au MTBE (éther de méthyle et de tert-butyle). Les phases organiques réunies sont lavées avec une solution de bicarbonate de sodium puis à l'eau salée.

Après séchage sur sulfate de magnésium, filtration sur papier et évaporation du solvant, on obtient un produit brut composé de deux isomères en proportion alpha/beta de 90:10, lorsque le déhydroherbac de départ est un mélange alpha/beta de 85:15. On obtient un produit brut composé de deux isomères en proportion alpha/beta de 60:40, lorsque le déhydroherbac de départ est un mélange alpha/beta de 60:40.

Dans les deux cas, le produit brut est distillé sous pression réduite.

T_{éb}. = 60 °C / 0,4 torr

Description olfactive 90:10 : boisé, pin, eucalyptus, très puissant.

Description olfactive 60:40 : boisé, camphré, très puissant.

Isomère alpha :

**¹H-NMR** (200 MHz, CDCl₃): δ (ppm) 0,76 (t,3H), 0,97 (s, 6H), 0,82-1,13 (m, 3H), 1,25 (s, 3H), 1,35-1,41 (m, 2H), 1,50-1,61 (m, 3H), 1,85-1,91 (m, 2H), 5,41 (s, 1H).

**¹³C-NMR** (50 MHz, CDCl3): δ (ppm) 8,2, 20,2, 27,2, 30,2, 30,3, 32,9, 37,5, 48,8, 54,3, 75,1, 130,5, 139,4.

**MS** [e/m (%)]: 182 (M+, 0,3), 154 (11), 153 (100), 109(16), 95(13), 81 (71), 57 (18), 55 (10),43 (48), 41 (10).

Isomère beta :

**¹H-NMR** (200 MHz, CDCl₃): δ (ppm) 0,76 (t,3H), 0,97 (s, 6H), 0,82-1,13 (m, 3H), 1,25 (s, 3H), 1,35-1,41 (m, 2H), 1,50-1,61 (m, 3H), 1,85-1,91 (m, 2H), 5,56-5,68 (m, 1H).

**¹³C-NMR** (50 MHz, CDCl₃); δ (ppm) 9,2, 12,3, 26,1, 29,2, 30,4, 32,1, 33,3, 41,3, 42,6, 53,7, 122,4, 128,3.

**MS** [e/m (%)]: 182 (M+, 5), 167 (10), 154 (25), 153 (33), 138 (34), 126 (25), 125 (29), 113 (20), 112 (10), 11 (12), 110 (57), 109 (16), 98 (11), 97 (40), 95 (19), 83 (100), 70 (16), 69 (73), 67 (14), 57 (22), 56 (33), 55 (75), 53 (13), 43 (18), 42 (12), 41 (51), 39 (18).

S'il est souhaité, des techniques bien connues de l'homme du métier, comme la distillation fractionnée, peuvent permettre de séparer ces deux isomères : 2-(3,3-diméthylcyclohex-1-ényl)butan-2-ol et 2-(5,5-diméthylcyclohex-1-ényl)butan-2-ol (respectivement désignés isomères alpha et beta, ci-avant). Par exemple, une distillation à l'aide d'une colonne avec garnissage métallique permet d'isoler dans un premier temps l'isomère alpha : sous 1 torr, il distille à 55 °C en tête de colonne. Puis, si la distillation est poursuivie, après quelques fractions de mélange alpha-beta, l'isomère beta pourra être isolé : sous 1 torr, il distille à 58 °C en tête de colonne.

De même, ces isomères peuvent être obtenus de manière sélective, par synthèse ; en remplaçant, dans le protocole précédant, l'hydroherbac par la 1-(3,3-diméthylcyclohex-1-ényl)éthanone ou la 1-(3,3-diméthylcylohex-6-ényl)éthanone.

### Exemple 2 : Préparation des 2-(3,3-diméthylcyclohex-1-ényl)pent-4-én-2-ol et 2-(5,5-diméthylcyclohex-1-ényl)pent-4-én-2-ol

Le protocole de l'exemple 1 est repris avec du chlorure d'allyle en remplacement du bromoéthane.

Le produit brut formé, composé de deux isomères en proportion alpha/beta de 88:12, est distillé sous pression réduite.
Téb. = 55 °C / 0,2 torr
Description olfactive : boisé, animal, moyennement puissant.
Isomère alpha:
   **¹H-NMR** (200 MHz, CDCl₃): δ (ppm) 0,97 (s, 6H), 1,26 (s, 3H), 1,30-1,41 (m, 2H), 1,59-1,65 (m, 2H), 1,75 (d, *J* = 2,2 Hz, 1H), 1,92 (td, *J* = 1,2 et 6,0 Hz, 2H), 2,16-2,45 (m, 2H), 5,04-5,13 (m, 2H), 5,42 (s, 1H), 5,61-5,79 (m, 1H).
   **¹³C-NMR** (50 MHz, CDCl₃): δ (ppm) 20,15, 25,07, 27,17, 30,33, 31,57, 37,09, 45,12, 73,92, 118,42, 130,68, 134,08, 139,56.
   **MS** [e/m (%)]: 194 (M+, 0), 154 (11), 153 (100), 109 (21), 95 (12), 91 (10), 81 (94), 67 (12), 43 (79), 41 (17).
Isomère beta :
   **¹H-NMR** (200 MHz, CDCl₃): δ (ppm) 0,91 (s, 6H), 1,26 (s, 3H), 1,30-1,41 (m, 2H), 1,59-1,65 (m, 2H), 1,75 (d, *J* = 2,2 Hz, 1H), 1,92 (td, *J* = 1,2 et 6,0 Hz, 2H), 2,16-2,45 (m, 2H), 5,04-5,13 (m, 2H), 5,42 (s, 1H), 5,61-5,79 (m, 1H).
   **¹³C-NMR** (50 MHz, CDCl3): δ (ppm) 23,00, 23,79, 28,12, 32,17, 34,88, 40,31, 50,19, 73,92, 118,64, 128,56, 134,08, 139,56.
   **MS** [e/m (%)]: 194 (M+, 0), 154 (10), 153 (100), 97 (34), 95 (40), 79 (12), 69 (14), 67 (11), 59 (10), 55 (13), 43 (20), 41 (23), 39 (11).

S'il est souhaité, 2-(3,3-diméthylcyclohex-1-ényl)pent-4-én-2-ol et 2-(5,5-diméthylcyclohex-1-ényl)pent-4-én-2-ol ainsi obtenus peuvent être séparés, par exemple, par distillation fractionnée. De même, ils peuvent être obtenus de manière sélective, par synthèse, à partir de la 1-(3,3-diméthylcyclohex-1-ényl)éthanone et la 1-(3,3-diméthylcylohex-6-ényl)éthanone.

### Exemple 3 : Préparation des 2-(3,3-diméthylcyclohex-1-ényl)pentan-2-ol et 2-(5,5-diméthylcyclohex-1-ényl)pentan-2-ol

Le protocole de l'exemple 1 est repris avec du 1-bromopropane en remplacement du bromoéthane.

Le produit brut obtenu, composé de deux isomères en proportion alpha/beta de 95:5, est distillé sous pression réduite.
Téb. = 53 °C / 0,2 torr
Description olfactive : boisé, animal, agreste, puissant.
Isomère alpha :
   **¹H-NMR** (200 MHz, CDCl3): δ (ppm) 0,85-1,07 (m, 6H), 0,97 (s, 6H), 1,25 (s, 3H), 1,35-1,50 (m, 4H), 1,54-1,70 (m, 4H), 1,88 (td, *J* = 1,2 et 6,0 Hz, 2H), 2,07-2,28 (m, 2H), 5,40 (s, 1 H).
   **¹³C-NMR** (50 MHz, CDCl₃): δ (ppm) 14,54, 17,21, 20,20, 24,98, 27,64, 30,25, 30,37, 31,58, 37,13, 42,88, 74,94, 130,19, 139,96.
   **MS** [e/m (%)]: 196 (M+, 0,3), 154 (10), 153 (100), 109 (15), 95 (12), 91 (11), 81 (67), 71 (14), 43 (47), 41 (12).
Isomère beta :
   **MS** [e/m (%)]: 196 (M+, 0,8), 154 (15), 153 (100), 97 (24), 95 (34), 69 (10), 43 (17), 41 (14).

S'il est souhaité, 2-(3,3-diméthylcyclohex-1-ényl)pentan-2-ol et 2-(5,5-diméthylcyclohex-1-ényl)pentan-2-ol ainsi obtenus peuvent être séparés, par exemple, par distillation fractionnée. De même, ils peuvent être obtenus de manière sélective, par synthèse, à partir de la 1-(3,3-diméthylcyclohex-1-ényl)éthanone et la 1-(3,3-diméthylcylohex-6-ényl)éthanone.

### Exemple 4 : Préparation des 2-(3,3-diméthylcyclohex-1-ényl)hexan-2-ol et 2-(5,5-diméthylcyclohex-1-ényl)hexan-2-ol

Le protocole de l'exemple 1 est repris avec du 1-bromobutane en remplacement du bromoéthane.

Le produit brut obtenu est composé de deux isomères en proportion alpha/beta de 92:8 lorsque le déhydroherbac de départ est un mélange alpha/beta de 85:15, et 80:20, lorsque le déhydroherbac de départ est un mélange alpha/beta de 60:40.
T_{éb}. = 57 °C / 0,2 torr
Description olfactive 92:8 : boisé, animal, légèrement frais, facette immortelle, moyennement puissant.
Description olfactive 80:20 : boisé, menthé, facette framboise, puissant.
Isomère alpha:
   **¹H-NMR** (200 MHz, CDCl₃): δ (ppm) 0,85-1,07 (m, 6H), 0,97 (s, 6H), 1,26 (s, 3H), 1,37-1,31 (m, 1H), 1,35-1,43 (m, 3H), 1,48-1,52 (m, 2H), 1,59-1,67 (m, 2H), 1,88 (td, *J* = 1,2 et 6,0 Hz, 2H), 2,08-2,28 (m, 2H), 5,40 (s, 1H).
   **¹³C-NMR** (50 MHz, CDCl₃): δ (ppm) 14,11, 20,21, 23,09, 24,97, 26,11, 27,65, 30,25, 30,37, 31,58, 37,14, 40,16, 74,91, 130,24, 139,94.
   **MS** [e/m (%)]: 210 (M+, 0,3),154 (11), 153 (100), 109 (12), 81 (57), 43 (32).
Isomère beta :
   **MS** [e/m (%)]: 210 (M+, 0,7), 153 (100), 97 (20), 95 (28), 43 (11), 41 (10).

S'il est souhaité, 2-(3,3-diméthylcyclohex-1-ényl)hexan-2-ol et 2-(5,5-diméthylcyclohex-1-ényl)hexan-2-ol ainsi obtenus peuvent être séparés, par exemple, par distillation fractionnée. De même, ils peuvent être obtenus de manière sélective, par synthèse, à partir de la 1-(3,3-diméthylcyclohex-1-ényl)éthanone et la 1-(3,3-diméthylcylohex-6-ényl)éthanone.

### Exemple 5 : Préparation des 2-(3,3-diméthylcyclohex-1-ényl)-4-méthylpentan-2-ol et 2-(5,5-diméthylcyclohex-1-ényl)-4-méthylpentan-2-ol

Le protocole de l'exemple 1 est repris avec du 1-bromo-2-méthylpropane en remplacement du bromoéthane.

Le produit brut obtenu, composé de deux isomères en proportion alpha/beta de 95:5, est distillé sous pression réduite.
T_{éb.} = 48 °C/0,2 torr
Description olfactive : boisé, frais, menthé, puissant.
Isomère alpha :
   **¹H-NMR** (200 MHz, CDCl₃): δ (ppm) 0,88 (d, *J* = 7,4 Hz, 3H), 0,92 (d, *J* = 7,4 Hz, 3H), 0,97 (s, 6H), 1,03-1,07 (m, 1H), 1,26 (s, 3H), 1,35-1,49 (m, 5H), 1,54-1,68 (m, 3H), 1,89 (td, *J* = 1,4 et 5,8 Hz, 2H), 5,44 (s, 1H).
   **¹³C-NMR** (50 MHz, CDCl₃): δ (ppm) 20,09, 24,32, 24,40, 25,25, 28,78, 29,90, 30,40, 31,52, 37,03, 48,88, 75,36, 129,91, 140,13.
   **MS** [e/m (%)]: 210 (M+, 0,1), 154 (12), 153 (100), 109 (15), 81 (59), 57 (10), 43 (37), 41 (11).
Isomère beta :
   **MS** [e/m (%)]: 210 (M+, 0,6), 154 (14), 153 (100), 97 (28), 95 (33), 69 (11), 57 (10), 43 (16), 41 (16).

S'il est souhaité, 2-(3,3-diméthylcyclohex-1-ényl)-4-méthylpentan-2-ol et 2-(5,5-diméthylcyclohex-1-ényl)-4-méthylpentan-2-ol ainsi obtenus peuvent être séparés, par exemple, par distillation fractionnée. De même, ils peuvent être obtenus de manière sélective, par synthèse, à partir de la 1-(3,3-diméthylcyclohex-1-ényl)éthanone et la 1-(3,3-diméthylcylohex-6-ényl)éthanone.

### Exemple 6 : Préparation des 2-(3,3-diméthylcyclohex-1-ényl)-3-méthylpentan-2-ol et 2-(5,5-diméthylcyclohex-1-ényl)-3-méthylpentan-2-ol

Le protocole de l'exemple 1 est repris avec du 2-bromobutane en remplacement du bromoéthane.

Le produit brut obtenu, composé de quatre isomères (deux diastéréomères alpha et diastéréomères beta) en proportion 47:40:7:6, est distillé sous pression réduite.
Description olfactive : boisé, gras, chloré, insecticide, moyennement puissant.
Téb. = 48 °C / 0,2 torr
4 isomères superposés :
   **¹H-NMR** (200 MHz, CDCl₃): δ (ppm) 0,82-0,93 (m, 6H), 0,97 (s, 3H), 1,19 et 1,21 (2 s, 3H), 1,40-1,71 (m, 7H), 1,88 (m, 2H).
   **¹³C-NMR** (50 MHz, CDCl₃, 47%): δ (ppm) 12,94, 13,20, 20,23, 23,51, 23,94, 24,85, 30,29, 31,64, 37,18, 41,44, 72,18, 130,63, 140,52.
   **¹³C-NMR** (50 MHz, CDCl₃, 40%): δ (ppm) 12,85, 12,99, 20,23, 23,77, 23,79, 24,89, 30,20, 30,39, 31,64, 31,18, 41,30, 72,18, 130,63, 140,52.
   **MS** [e/m (%)], isomère à 47% : 210 (M+, 0,1), 154(11), 153 (100), 109 (18), 95 (10), 81 (78), 43 (45), 41(12).
   **MS** [e/m (%)], isomère à 40% : 210 (M+, 0,2), 154 (10), 153 (100), 109 (20), 95 (11), 81 (80), 43 (46), 41 (12).
   **MS** [e/m (%)], isomère à 7% : 210 (M+, 0), 154 (11), 153 (100), 97 (30), 95 (38), 69 (11), 43 (14), 41 (16).
**MS** [e/m (%)], isomère à 6% : 210 (M+, 0), 154 (11), 153 (100), 97 (25), 95 (27), 43 (13), 41 (13).

S'il est souhaité, 2-(3,3-diméthylcyclohex-1-ényl)-3-méthylpentan-2-ol et 2-(5,5-diméthylcyclohex-1-ényl)-3-méthylpentan-2-ol ainsi obtenus peuvent être séparés, par exemple, par distillation fractionnée. De même, ils peuvent être obtenus de manière sélective, par synthèse, à partir de la 1-(3,3-diméthylcyclohex-1-ényl)éthanone et la 1-(3,3-diméthylcylohex-6-ényl)éthanone.

### Exemple 7 : Préparation des 2-(3,3-diméthylcyclohex-1-ényl)-5-méthylhexan-2-ol et 2-(5,5-diméthylcyclohex-1-ényl)-5-méthylhexan-2-ol

Le protocole de l'exemple 1 est repris avec du 3-méthyl-1-bromobutane en remplacement du bromoéthane.

Le produit brut obtenu, composé de deux isomères en proportion alpha/beta de 89:11, est distillé sous pression réduite.
T_{éb}. = 85 °C / 0,4 torr
Description olfactive : boisé, foin sec, miel, puissant.
Isomère alpha:
   **¹H-NMR** (200 MHz, CDCl₃): δ (ppm) 0,87 (d, *J* = 6,4 Hz, 6H), 0,97 (s, 6H), 1,02-1,09 (m, 2H), 1,26 (s, 3H), 1,35-1,62 (m, 8H), 1,88 (td, *J* = 1,0 et 5,8 Hz, 2H), 5,40 (s, 1H).
   **¹³C-NMR** (50 MHz, CDCl₃): δ (ppm) 20,19, 22,63, 22,70, 24,95, 27,70, 28,33, 30,26, 30,35, 31,59, 32,92, 37,13, 38,11, 74,92, 130,34, 139,83.
   **MS** [e/m (%)]: 224 (M+, 0,2), 154(11), 153 (100), 135 (10), 109 (13), 81 (64), 43 (42), 41 (11).
Isomère beta :
   **¹H-NMR** (200 MHz, CDCl₃): δ (ppm) 0,87 (d, *J* = 6,4 Hz, 6H), 0,97 (s, 6H), 1,02-1,09 (m, 2H), 1,26 (s, 3H), 1,35-1,62 (m, 8H), 1,88 (td, *J* = 1,0 et 5,8 Hz, 2H), 5,60-,66 (m, 1H).
   **MS** [e/m (%)]: 224 (M+, 0), 154 (17), 153 (100), 97 (26), 95 (30), 69 (12), 43 (23), 41 (11).

S'il est souhaité, 2-(3,3-diméthylcyclohex-1-ényl)-5-méthylhexan-2-ol et 2-(5,5-diméthylcyclohex-1-ényl)-5-méthylhexan-2-ol ainsi obtenus peuvent être séparés, par exemple, par distillation fractionnée. De même, ils peuvent être obtenus de manière sélective, par synthèse, à partir de la 1-(3,3-diméthylcyclohex-1-ényl)éthanone et la 1-(3,3-diméthylcylohex-6-ényl)éthanone.

### Exemple 8 : Préparation du 2-(3,3-diméthylcyclohexyl)butan-2-ol

Le protocole de l'exemple 1 est repris avec de l'herbac en remplacement du déhydroherbac.

Le produit brut obtenu, composé de trois isomères en proportion 38:37:25, est distillé sous pression réduite.
T_{éb}. = 59 °C/1,0 torr
Description olfactive : boisé, fruité, linalol, puissant.
3 isomères superposés :
   **¹H-NMR** (200 MHz, CDCl₃): δ (ppm) 0,85-0,93 (m, 12H), 1,03-1,13 (m, 4H), 1,32-1,59 (m, 8H). **¹³C-NMR** (50 MHz, CDCl₃, isomères majoritaires (38 et 37%)): δ (ppm) 7,53 et 7,77, 22,56 et 23,23, 23,41 et 23,58, 24,66 et 24,74, 26,54 et 27,10, 32,28 et 32,39, 33,78, 34,59 et 35,66, 39,17 et 39,23, 41,66 et 41,80, 74,37 et 74,44.
   **MS** [e/m (%)], isomère à 3 8% : 224 (M+, 0), 155 (12), 95 (11), 73 (100), 72 (16), 69 (14), 55 (19), 43 (15), 41 (11).
   **MS** [e/m (%)], isomère à 37% : 224 (M+, 0), 155 (13), 137 (10), 95 (12), 73 (100), 72 (17), 69 (15), 55 (20), 43 (15), 41 (12).
   **MS** [e/m (%)], isomère à 25% : 224 (M+, 0), 155 (36), 137 (31), 113 (100), 111 (15), 99 (50), 95 (17), 86 (18), 85 (10), 83 (12), 81 (14), 73 (11), 71 (10), 69 (24), 57 (34), 56 (15), 55 (29), 43 (28), 41 (25).

### Exemple 9 : Préparation du 2-(3,3-diméthylcyclohexyl)pent-4-én-2-ol

Le protocole de l'exemple 1 est repris avec du chlorure d'allyle en remplacement du bromoéthane, et de l'herbac en remplacement du déhydroherbac.

Le produit brut obtenu, composé de trois isomères en proportion 42:41:17, est distillé sous pression réduite.
T_{éb.} = 72 °C / 0,4 torr
Description olfactive : boisé, fleuri, vert, puissant.
3 isomères superposés:
   **¹H-NMR** (200 MHz, CDCl₃): δ (ppm) 0,88-0,93 (3s, 6H), 0,82-1,00 (m, 2H), 1,08-1,09 (3s, 3H), 1,32-1,64 (m, 7H), 2,12-2,32 (m, 2H), 5,06-5,17 (m, 2H), 5,78-5,95 (m, 1H).
   **¹³C-NMR** (50 MHz, CDCl₃, isomères majoritaires (42 et 41%)): δ (ppm) 22,50, 23,50 et 23,86, 24,64 et 24,71, 26,57 et 27,23, 30,84 et 30,94, 33,75, 39,18 et 39,24, 39,71 et 40,66, 42,51 et 42,55, 44,36 et 44,43, 73,77 et 73,89, 118,59 et 118,66, 134,03 et 134,16.
   **MS** [e/m (%)], isomère à 42% : 196 (M+, 0), 156 (11), 155 (100), 137 (65), 111 (53), 95 (51), 85 (46), 84 (17), 81 (38), 69 (59), 67 (13), 55 (31), 45 (13), 43 (98), 41 (35), 39 (11).
   **MS** [e/m (%)], isomère à 41% : 196 (M+, 0), 156 (12), 155 (97), 137 (61), 111 (52), 97 (10), 95 (51), 85 (45), 84 (18), 81 (37), 69 (60), 67 (15), 55 (32), 45 (12), 43 (100), 41 (33), 39 (13).
   **MS** [e/m (%)], isomère à 17% : 196 (M+, 0,7), 156 (10), 155 (100), 138 (10), 137 (80), 125 (27), 111 (40), 98 (16), 97 (22), 95 (45), 85 (14), 84 (12), 83 (97), 81 (39), 69 (80), 59 (21), 57 (23), 56 (21), 55 (65), 53 (12), 43 (56), 42 (12), 41 (65), 39 (20).

### Exemple 10 : Préparation du 2-(3,3-diméthylcyclohexyl)pentan-2-ol

Le protocole de l'exemple 1 est repris avec du 1-bromopropane en remplacement du bromoéthane, et de l'herbac en remplacement du déhydroherbac.

Le produit brut obtenu, composé de trois isomères en proportion 79:19:2, est distillé sous pression réduite.
T_{éb}. = 60 °C / 0,2 torr
Description olfactive : boisé, frais, linalol, liquoreux, puissant.
3 isomères superposés :
   **¹H-NMR** (200 MHz, CDCl₃): δ (ppm) 0,89-0,92 (2s et 1t superposés, 9H), 1,03-1,09 (m, 2H), 1,07-1,09 (2s, 3H), 1,31-1,83 (m, 11H).
   **¹³C-NMR** (50 MHz, CDCl₃, isomères à 79%): δ (ppm) 14,77, 16,64, 22,53, 24,12, 24,67, 27,14, 30,85, 33,78, 39,24, 39,76, 42,41, 42,48, 74,44.
   **¹³C-NMR** (50 MHz, CDCl₃, isomères à 19%): δ (ppm) 16,44, 16,68, 22,56, 23,89, 24,74, 26,60, 30,92, 33,78, 39,18, 40,59, 42,30, 42,34, 74,33.
   **MS** [e/m (%)], isomère à 79% : 198 (M+, 0), 155 (16), 137 (11), 95 (11), 87 (100), 86 (12), 69 (18), 55 (11), 45 (21), 43 (15), 41 (13).
   **MS** [e/m (%)], isomère à 19% : 198 (M+, 0), 155 (16), 137 (10), 95 (10), 87 (100), 86 (11), 69 (19), 55 (10), 45 (20), 43 (15), 41 (12).
   **MS** [e/m (%)], isomère à 2% : 198 (M+, 0), 155 (38), 137 (32), 127 (100), 113 (48), 111 (14), 100 (12), 95 (14), 83 (13), 81 (13), 71 (24), 69 (24), 57 (16), 56 (12), 55 (26), 43 (34), 41 (25).

### Exemple 11 : Préparation du 2-(3,3-diméthylcyclohexyl)hexan-2-ol

Le protocole de l'exemple 1 est repris avec du 1-bromobutane en remplacement du bromoéthane, et de l'herbac en remplacement du déhydroherbac.

Le produit brut obtenu, composé de trois isomères en proportion 77:21:2, est distillé sous pression réduite.
T_{éb}. = 68 °C / 0,2 torr
Description olfactive : boisé, animal, moyennement puissant.
3 isomères superposés :
   **¹H-NMR** (200 MHz, CDCl₃): δ (ppm) 0,89-0,92 (2s et lt superposés, 9H), 1,03-1,09 (m, 2H), 1,08-1,09 (2s, 3H), 1,31-1,83 (m, 13H).
   **¹³C-NMR** (50 MHz, CDCl₃, isomères à 77%): δ (ppm) 14,17, 16,64, 22,39, 24,15, 24,68, 25,60, 27,15, 30,93, 33,78, 39,18, 39,68, 39,79, 42,34, 74,30.
   **¹³C-NMR** (50 MHz, CDCl₃, isomères à 21%): δ (ppm) 16,69, 22,57, 23,95, 24,76, 25,47, 26,58, 30,85, 33,78, 39,23, 39,75, 40,57, 42,23, 74,40.
   **MS** [e/m (%)], isomère à 77% : 212 (M+, 0), 155 (20), 137 (11), 101 (100), 95 (10), 69 (13), 55 (14), 45 (10), 43 (12), 41 (11).
   **MS** [e/m (%)], isomère à 21% : 212 (M+, 2), 155 (48), 141 (100), 137 (33), 127 (41), 111 (13), 95 (15), 85 (16), 83 (11), 81 (13), 71 (12), 69 (20), 57 (19), 55 (23), 43 (20), 41 (23).
   **MS** [e/m (%)], isomère à 2% : 212 (M+, 1), 156 (11), 155 (57), 141 (44), 137 (32), 127 (21), 111 (11), 101 (100), 100 (13), 95 (23), 83 (10), 81 (20), 71 (15), 69 (17), 55 (37), 53 (10), 45 (12), 43 (28), 41 (21).

### Exemple 12 : Préparation du 2-(3,3-diméthylcyclohexyl)-4-méthylpentan-2-ol

Le protocole de l'exemple 1 est repris avec du 1-bromo-2-méthylpropane en remplacement du bromoéthane, et de l'herbac en remplacement du déhydroherbac.

Le produit brut obtenu, composé de trois isomères en proportion 85:14:1, est distillé sous pression réduite.
T_{éb.} = 58 °C / 0,2 torr
Description olfactive : boisé, frais, un peu citronnelle et banane, moyennement puissant.
**¹H-NMR** (200 MHz, CDCl₃): δ (ppm) 0,81-1,06 (m, 2H), 0,89-0,98 (2s et 2d superposés, 12H), 1,09-1,10 (2s, 3H), 1,34-1,85 (m, 10H).
**¹³C-NMR** (50 MHz, CDCl₃, isomères à 85%): δ (ppm) 22,54, 23,73, 24,22, 24,74, 24,89, 25,02, 26,73, 30,96, 33,77, 39,28, 40,72, 43,47, 48,24, 75,09.
**¹³C-NMR** (50 MHz, CDCl₃, isomères à 14%): δ (ppm) 23,64, 23,82, 24,37, 24,82, 25,09, 27,37, 30,89, 33,80, 39,23, 39,88, 43,51, 48,24, 74,98.
**MS** [e/m (%)], isomère à 85% : 212 (M+, 0), 155 (26), 137 (13), 101 (100), 95 (11), 69 (15), 59 (14), 57 (21), 55 (11),43 (19), 41 (15).
**MS** [e/m (%)], isomère à 14% : 212 (M+, 2), 155 (40), 141 (100), 137 (28), 127 (33), 111 (14), 95 (15), 85 (22), 83 (12), 81 (16), 71 (11), 69 (23), 57 (22), 56 (11), 55 (24), 43 (25), 41 (28).
**MS** [e/m (%)], isomère à 1% : 212 (M+, 0), 155 (46), 137 (24), 111 (10), 101 (100), 95 (23), 85 (11), 83 (10), 81 (12), 69 (14), 59 (13), 58 (11), 57 (22), 45 (10), 43 (26), 41 (19).

### Exemple 13 : Préparation du 2-(3,3-diméthylcyclohexyl)-3-méthylpentan-2-ol

Le protocole de l'exemple 1 est repris avec du 2-bromobutane en remplacement du bromoéthane, et de l'herbac en remplacement du déhydroherbac.

Le produit brut obtenu, composé de cinq isomères en proportion 26:25:24:20:5, est distillé sous pression réduite.
T_{éb.} = 64 °C / 0,2 torr
Description olfactive : boisé, armoise, savoureux, subtile.
5 isomères superposés :
   **¹H-NMR** (200 MHz, CDCl₃): δ (ppm) 0,80-1,05 (m, 4H), 0,89-0,95 (m, 12H), 0,99-1,01 (s superposés, 3H), 1,31-1,82 (m, 9H).
   **MS** [e/m (%)], isomère à 26% : 212 (M+, 0,2), 155 (48), 141 (11), 137 (33), 111 (21), 101 (100), 95 (24), 81 (15), 72 (10), 69 (26), 59 (14), 57 (20), 55 (21), 45 (21), 43 (26), 41 (22).
   **MS** [e/m (%)], isomère à 25% : 212 (M+, 0,1), 155 (42), 137 (26), 111 (20), 101 (100), 95 (19), 81 (13), 69 (23), 59 (13), 57 (15), 55 (16), 45 (19), 43 (19), 41 (17).
   **MS** [e/m(%)], isomère à 24% : 212(M+, 0), 155 (36), 137 (26), 111 (16), 101 (100), 95 (18), 81 (11), 69 (60), 59 (11), 57 (15), 55 (15), 45 (20), 43 (17), 41 (16).
   **MS** [e/m (%)], isomère à 20% : 212 (M+, 0), 155 (40), 137 (25), 111 (21), 101 (100), 95 (21), 81 (13), 69 (22), 59 (11), 57 (15), 55 (16), 45 (19),43 (19), 41 (18).
   **MS** [e/m (%)], isomère à 5% : 212 (M+, 1), 155 (57), 141 (100), 137 (59), 127 (12), 111 (20), 97 (20), 95 (27), 85 (15), 83 (24), 81 (19), 71 (18), 69 (36), 67 (14), 59 (15), 57 (39), 56 (13), 55 (39), 43 (41), 41 (33).

### Exemple 14 : Préparation du 2-(3,3-diméthylcyclohexyl)-5-méthylhexan-2-ol

Le protocole de l'exemple 1 est repris avec du 3-méthyl-1-bromobutane en remplacement du bromoéthane, et de l'herbac en remplacement du déhydroherbac.

Le produit brut obtenu, composé de trois isomères en proportion 46:43:11, est distillé sous pression réduite.
T_{éb}. = 71 °C/0,4 torr
Description olfactive : boisé, pin, frais, propre, puissant.
3 isomères superposés :
   **¹H-NMR** (200 MHz, CDCl₃): δ (ppm) 0,88-0,94 (s superposés, 12H), 0,87-1,00 (m, 2H), 1,04-1,12 (m, 1H), 1,07-1,08 (2s, 3H), 1,17-1,26 (m, 3H), 1,31-1,32 (m, 1H), 1,39-1,53 (m, 7H), 1,57-1,83 (m, 1H).
   **¹³C-NMR** (50 MHz, CDCl₃, isomères majoritaires (46 et 43%)): δ (ppm) 22,54 et 22,57, 22,73, 24,01 et 24,17, 24,69 et 24,77, 26,55 et 27,14, 28,65 et 28,74, 32,20 et 33,78, 32,38 et 32,78, 34,75, 37,71 et 37,79, 39,17 et 39,21, 39,72 et 40,57, 42,07 et 42,20, 74,29 et 74,38.
   **MS** [e/m (%)], isomère à 46% : 226 (M+, 0), 155 (38), 137 (20), 115 (100), 111 (14), 97 (54), 95 (15), 81 (13), 71 (12), 69 (25), 57 (11), 55 (32), 43 (26), 41 (19).
   **MS** [e/m (%)], isomère à 43% : 226 (M+, 0,4), 156 (10), 155 (100), 137 (24), 95 (12), 83 (13), 81 (15), 69 (18), 57 (13), 55 (16), 43 (17), 41 (15).
   **MS** [e/m (%)], isomère à 11% : 226 (M+, 4), 155 (100), 137 (30), 127 (10), 115 (54), 111 (11), 97 (51), 95 (18), 81 (19), 79 (11), 72 (11), 69 (38), 67 (15), 58 (14), 57 (13), 56 (10), 55 (27), 45 (12), 43 (32), 41 (30).

### Exemple 15 : Compositions parfumantes A, B, C et D incorporant un des dérivés de formule générale I obtenus selon les exemples 8, 9 et 14.

Un accord patchouli-ambré A, puis le même accord comprenant le 2-(3,3-diméthylcyclohexyl)butan-2-ol obtenu selon l'exemple 8 pour donner l'accord B, puis le même accord comprenant le 2-(3,3-diméthylcyclohexyl)pent-4-én-2-ol obtenu selon l'exemple 9 pour donner l'accord C, puis le même accord comprenant le 2-(3,3-diméthylcyclohexyl)-5-méthylhexan-2-ol obtenu selon l'exemple 14 pour donner l'accord D.

**Tableau : Formulations A, B, C et D pour eau de toilette masculine**

| **Ingrédients** | **Accord A** | **Accord B** | **Accord C** | **Accord D** |
|---|---|---|---|---|
| ESSENCE DE BERGAMOTE | 0,40 | 0,40 | 0,40 | 0,40 |
| VANILLINE | 3,85 | 3,85 | 3,85 | 3,85 |
| ESSENCE DE PATCHOULI | 38,45 | 38,45 | 38,45 | 38,45 |
| THYMOL | 0,04 | 0,04 | 0,04 | 0,04 |
| ABSOLUE DE CISTE | 0,38 | 0,38 | 0,38 | 0,38 |
| ETHYLVANILLINE | 0,62 | 0,62 | 0,62 | 0,62 |
| FIXOLlDE® | 0,77 | 0,77 | 0,77 | 0,77 |
| MUSC CETONE | 1,54 | 1,54 | 1,54 | 1,54 |
| DIPROPYLENE GLYCOL | 53,95 | 50,10 | 50,10 | 50,10 |
| **2-(3,3-diméthylcyclohexyl)butan-2-ol** | **0,00** | **3,85** | **0,00** | **0,00** |
| **2-(3,3-diméthylcyclohexyl)pent-4-én-2-ol** | **0,00** | **0,00** | **3,85** | **0,00** |
| **2-(3,3-diméthylcyclohexyl)-5-méthylhexan-2-ol** | **0,00** | **0,00** | **0,00** | **3,85** |

Les formulations ainsi obtenues et détaillées ci-dessus sont utilisées comme bases parfumantes : chacune d'elles est incorporée à 10% en poids dans une base alcoolique en vue de préparer une eau de toilette masculine prête à l'emploi.

L'évaluation olfactive et comparative des accords A, B, C et D à 10% en poids en base alcoolique montre que l'ajout du 2-(3,3-diméthylcyclohexyl)butan-2-ol à hauteur de 3,85% en poids dans l'accord B accentue très notablement la facette moisie, patchouli par rapport à l'accord A. L'ajout du 2-(3,3-diméthylcyclohexyl)pent-4-én-2-ol à hauteur de 3,85% en poids dans l'accord C renforce également la facette patchouli ainsi que la note vanillée par rapport à l'accord A. Enfin, l'ajout du 2-(3,3-diméthylcyclohexyl)-5-méthylhexan-2-ol à hauteur de 3,85% en poids dans l'accord D apporte une richesse à l'accord avec des notes fruitées, fumées, naturelles de feuilles séchées par rapport à l'accord A.

### Exemple 16 : Compositions parfumantes E, F, G et H incorporant un des dérivés de formule générale I obtenus selon les exemples 8 et 9.

Un accord chypre fruité-boisé E, puis le même accord comprenant le 2-(3,3-diméthylcyclohexyl)butan-2-ol obtenu selon l'exemple 8 pour donner les accords F et G, puis le même accord comprenant le 2-(3,3-diméthylcyclohexyl)pent-4-én-2-ol est obtenu selon l'exemple 9 pour donner l'accord H.

**Tableau : Formulations E, F, G et H D pour eau de toilette féminine**

| **Ingrédients** | **Accord E** | **Accord F** | **Accord G** | **Accord H** |
|---|---|---|---|---|
| ESSENCE DE BERGAMOTE | 0,77 | 0,77 | 0,77 | 0,77 |
| ESSENCE DE PATCHOULI | 3,85 | 3,85 | 3,85 | 3,85 |
| ETHYLVANILLINE | 0,77 | 0,77 | 0,77 | 0,77 |
| CEDRENE TEXAS | 0,77 | 0,77 | 0,77 | 0,77 |
| METHYLIONANTHEME SUPER | 0,23 | 0,23 | 0,23 | 0,23 |
| ETHYLE LINALOL | 3,85 | 3,85 | 3,85 | 3,85 |
| FLOROL® | 4,62 | 4,62 | 4,62 | 4,62 |
| HYDROXYCITRONELLAL | 3,85 | 3,85 | 3,85 | 3,85 |
| GAMMA UNDECALACTONE | 1,54 | 1,54 | 1,54 | 1,54 |
| BETA IONONE | 6,92 | 6,92 | 6,92 | 6,92 |
| DIHYDROJASMONATE DE METHYLE | 21,54 | 21,54 | 21,54 | 21,54 |
| HELIONAL® | 1,23 | 1,23 | 1,23 | 1,23 |
| EVERNYL® | 0,15 | 0,15 | 0,15 | 0,15 |
| LYRAL® | 5,38 | 5,38 | 5,38 | 5,38 |
| CASHMERAN® | 0,38 | 0,38 | 0,38 | 0,38 |
| DIPROPYLENE GLYCOL | 44,15 | 43,38 | 40,30 | 40,30 |
| **2-(3,3-diméthylcyclohexvl)butan-2-ol** | **0,00** | **0,77** | **0,00** | **0,00** |
| **2-(3,3-diméthylcyclohexyl)butan-2-ol** | **0,00** | **0,00** | **3,85** | **0,00** |
| **2-(3,3-diméthylcyclohexyl)pent-4-én-2-ol** | **0,00** | **0,00** | **0,00** | **3,85** |

Les formulations décrites ci-dessus sont utilisées comme bases parfumantes : chacune d'elles est incorporée à 12% en poids dans une eau de toilette féminine prête à l'emploi :
L'évaluation olfactive et comparative des accords E, F, G et H à 12% en poids en base alcoolique montre que l'ajout du 2-(3,3-diméthylcyclohexyl)butan-2-ol à hauteur de 0,77% en poids dans l'accord F est très apprécié car il accentue la facette fumée, fruitée par rapport à l'accord E. L'ajout du 2-(3,3-diméthylcyclohexyl)butan-2-ol à hauteur de 3,85% en poids dans l'accord G appuie très fortement la facette moisie, patchouli par rapport à l'accord E. Enfin l'ajout du 2-(3,3-diméthyleyclohexyl)pent-4-én-2-ol 2 à hauteur de 3,85% en poids dans l'accord H apporte une un côté rond et chaud par rapport à l'accord E.

## Revendications

1. Composé de formule générale : 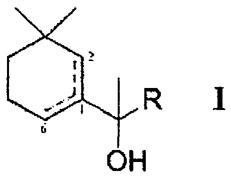 dans laquelle :
- le cycle à 6 atomes de carbone est saturé ou présente une double liaison entre les carbones C1 et C2 ou entre les carbones C1 et C6,
- R est choisi parmi un groupe alkyle en C₂-C₅ ou alcényle en C₂-C₅.

2. Composé selon la revendication 1, **caractérisé en ce que** le cycle à 6 atomes de carbone est saturé.

3. Composé selon la revendication 1, **caractérisé en ce que** le cycle à 6 atomes de carbone présente une double liaison entre les carbones C1 et C2 ou entre les carbones C1 et C6,

4. Composé selon l'une quelconque des revendications 1 à 3, **caractérisé en ce que** R est choisi parmi: -CH₂CH₃, -CH₂CH₂=CH₂, -CH₂CH₂CH₃, -CH₂CH₂CH₂CH₃, -CH₂CH(CH₃)₂, -CH(CH₃)CH₂CH₃ et -CH₂CH₂CH(CH₃)₂

5. Composé selon l'une quelconque des revendications 1 à 4, **caractérisé en ce qu'**il est choisi parmi :
- 2-(3,3-diméthylcyclohex-1-ényl)butan-2-ol
- 2-(5,5-diméthylcyclohex-1-ényl)butan-2-ol
- 2-(3,3-diméthylcyclohex-1-ényl)pent-4-en-2-ol
- 2-(5,5-diméthylcyclohex-1-ényl)pent-4-en-2-ol
- 2-(3,3-diméthylcyclohex-1-ényl)pentan-2-ol
- 2-(5,5-diméthylcyclohex-1-ényl)pentan-2-ol
- 2-(3,3-diméthylcyclohex-1-ényl)hexan-2-ol
- 2-(5,5-diméthylcyclohex-1-ényl)hexan-2-ol
- 2-(3,3-diméthylcyclohex-1-ényl)-4-méthylpentan-2-ol
- 2-(5,5-diméthylcyclohex-1 -ényl)-4-méthylpentan-2-ol
- 2-(3,3-diméthylcyclohex-1-ényl)-3-méthylpentan-2-ol
- 2-(5,5-diméthylcyclohex-1-ényl)-3-méthylpentan-2-ol
- 2-(3,3-diméthylcyclohex-1-ényl)-5-méthylhexan-2-ol
- 2-(5,5-diméthylcyclohex-1-ényl)-5-méthylhexan-2-ol
- 2-(3,3-diméthylcyclohexyl)butan-2-ol
- 2-(3,3-diméthylcyclohexyl)pent-4-en-2-ol
- 2-(3,3-diméthylcyclohexyl)pentan-2-ol
- 2-(3,3-diméthylcyclohexyl)hexan-2-ol
- 2-(3,3-diméthylcyclohexyl)-4-méthylpentan-2-ol
- 2-(3,3-diméthylcyclohexyl)-5-méthylhexan-2-ol

6. Composition parfumée comprenant au moins un composé selon l'une quelconque des revendications 1 à 5.

7. Composition selon la revendication 6, **caractérisée en ce qu'**il s'agit d'une composition de parfum.

8. Composition selon la revendication 7, **caractérisée en ce qu'**elle est choisie parmi : un concentré parfumant, une eau de Cologne, une eau de toilette, un parfum.

9. Composition selon la revendication 6, **caractérisée en ce qu'**il s'agit d'une composition cosmétique.

10. Composition selon la revendication 9, **caractérisée en ce qu'**elle est choisie parmi : une crème pour le visage et/ou le corps, une poudre de talc, une huile pour cheveux ou pour le corps, un shampooing, une lotion capillaire, des sels de bain, une huile de bain, un gel de douche, un gel de bain, un savon, un antiperspirant, un déodorant, une lotion, une crème de rasage, un savon de rasage, une pâte dentifrice, une pommade.

11. Composition selon la revendication 6, **caractérisée en ce qu'**il s'agit d'un produit d'entretien.

12. Composition selon la revendication 11, **caractérisée en ce qu'**elle est choisie parmi : un assouplissant, un détergent, une lessive, un désodorisant d'intérieur, un parfum d'ambiance.

13. Composition selon la revendication 6, **caractérisée en ce qu'**il s'agit d'une composition dite intermédiaire, destinée à être utilisée pour la préparation d'une composition selon l'une quelconque des revendications 7 à 12.

## Patentansprüche

1. Eine Verbindung mit der allgemeinen Formel: 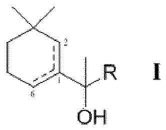 wobei:
- der Ring aus 6 Kohlenstoffatomen gesättigt ist oder eine Doppelbindung zwischen den Kohlenstoffen C1 und C2 oder zwischen den Kohlenstoffen C1 und C6 aufweist,
- R aus einer C₂-C₅-Alkylgruppe oder C₂-C₅-Alkenylgruppe ausgewählt ist.

2. Verbindung gemäß Anspruch 1, **dadurch gekennzeichnet, dass** der Ring aus 6 Kohlenstoffatomen gesättigt ist.

3. Verbindung gemäß Anspruch 1, **dadurch gekennzeichnet, dass** der Ring aus 6 Kohlenstoffatomen eine Doppelbindung zwischen den Kohlenstoffen C1 und C2 oder zwischen den Kohlenstoffen C1 und C6 aufweist.

4. Verbindung gemäß einem der Ansprüche 1 bis 3, **dadurch gekennzeichnet, dass** R ausgewählt ist aus: -CH₂CH₃, -CH₂CH₂=CH₂, -CH₂CH₂CH₃, -CH₂CH₂CH₂CH₃, -CH₂CH(CH₃)₂, -CH(CH₃)CH₂CH₃ und -CH₂CH₂CH(CH₃)₂.

5. Verbindung gemäß einem der Ansprüche 1 bis 4, **dadurch gekennzeichnet, dass** sie ausgewählt ist aus:
- 2-(3,3-Dimethylcyclohex-1-enyl)butan-2-ol
- 2-(5,5-Dimethylcyclohex-1-enyl)butan-2-ol
- 2-(3,3-Dimethylcyclohex-1-enyl)pent-4-en-2-ol
- 2-(5,5-Dimethylcyclohex-1-enyl)pent-4-en-2-ol
- 2-(3,3-Dimethylcyclohex-1-enyl)pentan-2-ol
- 2-(5,5-Dimethylcyclohex-1-enyl)pentan-2-ol
- 2-(3,3-Dimethylcyclohex-1-enyl)hexan-2-ol
- 2-(5,5-Dimethylcyclohex-1-enyl)hexan-2-ol
- 2-(3,3-Dimethylcyclohex-1-enyl)-4-methylpentan-2-ol
- 2-(5,5-Dimethylcyclohex-1-enyl)-4-methylpentan-2-ol
- 2-(3,3-Dimethylcyclohex-1-enyl)-3-methylpentan-2-ol
- 2-(5,5-Dimethylcyclohex-1-enyl)-3-methylpentan-2-ol
- 2-(3,3-Dimethylcyclohex-1-enyl)-5-methylhexan-2-ol
- 2-(5,5-Dimethylcyclohex-1-enyl)-5-methylhexan-2-ol
- 2-(3,3-Dimethylcyclohexyl)butan-2-ol
- 2-(3,3-Dimethylcyclohexyl)pent-4-en-2-ol
- 2-(3,3-Dimethylcyclohexyl)pentan-2-ol
- 2-(3,3-Dimethylcyclohexyl)hexan-2-ol
- 2-(3,3-Dimethylcyclohexyl)-4-methylpentan-2-ol
- 2-(3,3-Dimethylcyclohexyl)-5-methylhexan-2-ol

6. Eine parfümierte Zusammensetzung, beinhaltend mindestens eine Verbindung gemäß einem der Ansprüche 1 bis 5.

7. Zusammensetzung gemäß Anspruch 6, **dadurch gekennzeichnet, dass** sie eine Parfümzusammensetzung ist.

8. Zusammensetzung gemäß Anspruch 7, **dadurch gekennzeichnet, dass** sie ausgewählt ist aus: einem parfümierenden Konzentrat, einem Kölnischwasser, einem Eau de Toilette, einem Parfüm.

9. Zusammensetzung gemäß Anspruch 6, **dadurch gekennzeichnet, dass** sie eine kosmetische Zusammensetzung ist.

10. Zusammensetzung gemäß Anspruch 9, **dadurch gekennzeichnet, dass** sie ausgewählt ist aus: einer Gesichtscreme und/oder Körpercreme, einem Talkum, einem Haaröl oder Körperöl, einem Shampoo, einer Haarlotion, Badesalzen, einem Badeöl, einem Duschgel, einem Badegel, einer Seife, einem Antischweißmittel, einem Deodorant, einer Lotion, einer Rasiercreme, einer Rasierseife, einer Zahnpasta, einer Salbe.

11. Zusammensetzung gemäß Anspruch 6, **dadurch gekennzeichnet, dass** sie ein Pflegeprodukt ist.

12. Zusammensetzung gemäß Anspruch 11, **dadurch gekennzeichnet, dass** sie ausgewählt ist aus: einem Weichspüler, einem Detergens, einem Waschmittel, einem Raum-Geruchsneutralisierer, einem Lufterfrischer.

13. Zusammensetzung gemäß Anspruch 6, **dadurch gekennzeichnet, dass** sie eine sogenannte intermediäre Zusammensetzung ist, die zur Verwendung bei der Herstellung einer Zusammensetzung gemäß einem der Ansprüche 7 bis 12 bestimmt ist.

## Claims

1. A compound of general formula: 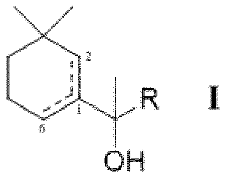 in which:
- the ring with 6 carbon atoms is saturated or has a double bond between carbons C1 and C2 or between carbons C1 and C6,
- R is selected from a C₂-C₅ alkyl or C₂-C₅ alkenyl group.

2. The compound according to claim 1, **characterised in that** the ring with 6 carbon atoms is saturated.

3. The compound according to claim 1, **characterised in that** the ring with 6 carbon atoms has a double bond between carbons C1 and C2 or between carbons C1 and C6.

4. The compound according to any one of claims 1 to 3, **characterised in that** R is selected from: -CH₂CH₃, -CH₂CH₂=CH₂, -CH₂CH₂CH₃, -CH₂CH₂CH₂CH₃, -CH₂CH(CH₃)₂, -CH(CH₃)CH₂CH₃ and -CH₂CH₂CH(CH₃)₂.

5. The compound according to any one of claims 1 to 4, **characterised in that** it is selected from:
- 2-(3,3-dimethylcyclohex-1-enyl)butan-2-ol
- 2-(5,5-dimethylcyclohex-1-enyl)butan-2-ol
- 2-(3,3-dimethylcyclohex-1-enyl)pent-4-en-2-ol
- 2-(5,5-dimethylcyclohex-1-enyl)pent-4-en-2-ol
- 2-(3,3-dimethylcyclohex-1-enyl)pentan-2-ol
- 2-(5,5-dimethylcyclohex-1-enyl)pentan-2-ol
- 2-(3,3-dimethylcyclohex-1-enyl)hexan-2-ol
- 2-(5,5-dimethylcyclohex-1-enyl)hexan-2-ol
- 2-(3,3-dimethylcyclohex-1-enyl)-4-methylpentan-2-ol
- 2-(5,5-dimethylcyclohex-1-enyl)-4-methylpentan-2-ol
- 2-(3,3-dimethylcyclohex-1-enyl)-3-methylpentan-2-ol
- 2-(5,5-dimethylcyclohex-1-enyl)-3-methylpentan-2-ol
- 2-(3,3-dimethylcyclohex-1-enyl)-5-methylhexan-2-ol
- 2-(5,5-dimethylcyclohex-1-enyl)-5-methylhexan-2-ol
- 2-(3,3-dimethylcyclohexyl)butan-2-ol
- 2-(3,3-dimethylcyclohexyl)pent-4-en-2-ol
- 2-(3,3-dimethylcyclohexyl)pentan-2-ol
- 2-(3,3-dimethylcyclohexyl)hexan-2-ol
- 2-(3,3-dimethylcyclohexyl)-4-methylpentan-2-ol
- 2-(3,3-dimethylcyclohexyl)-5-methylhexan-2-ol

6. A perfumed composition comprising at least one compound according to any one of claims 1 to 5.

7. The composition according to claim 6, **characterised in that** it is a perfume composition.

8. The composition according to claim 7, **characterised in that** it is selected from: a perfuming concentrate, an eau de Cologne, an eau de toilette, a perfume.

9. The composition according to claim 6, **characterised in that** it is a cosmetic composition.

10. The composition according to claim 9, **characterised in that** it is selected from: a face and/or body cream, a talcum powder, an oil for the hair or for the body, a shampoo, a hair lotion, bath salts, a bath oil, a shower gel, a bath gel, a soap, an antiperspirant, a deodorant, a lotion, a shaving cream, a shaving soap, a toothpaste, an ointment.

11. The composition according to claim 6, **characterised in that** it is a cleaning product.

12. The composition according to claim 11, **characterised in that** it is selected from: a softener, a detergent, a laundry detergent, an air-freshener, a room odoriser.

13. The composition according to claim 6, **characterised in that** it is a so-called intermediate composition, intended to be used for the preparation of a composition according to any one of claims 7 to 12.
